# EUROPEAN PATENT APPLICATION

(11) **EP 0 839 509 A1**
(43) Date of publication of application: **06.05.1998**
(21) Application number: 97307177.2
(22) Date of filing: 16.09.1997
(51) Int. Cl.: A61G 13/10, A47C 21/04

(54) **Mattress assemblies and patient support tables**

(30) Priority: 31.10.1996 GB 9622690
(71) Applicant: Smiths Industries Public Limited Company, London, NW11 8DS (GB)
(72) Inventor: Hannant, Keith, Rustington, West Sussex BN16 2QN (GB)
(74) Representative: Flint, Jonathan McNeill

(57) **Abstract**

A mattress 61 for a surgical operating table is made of foam 62 with an embedded tube 64, which follows a serpentine path across the mattress between an inlet 65 and outlet 66. The inlet and outlet of the tube 64 are connected to the outlet and inlet respectively of a heat exchanger 71 by which heated or cooled air can be supplied to warm or cool the mattress 61 and hence the patient.

## Description

This invention relates to mattress assemblies of the kind including a foam mattress for a patient suport table.

Modern surgical techniques often require the patient's body temperature to be controlled during surgery. The temperature may need to be raised or lowered from normal body temperature. One way of controlling temperature is by means of a heated blanket placed on top of the patient but, in many cases, this is not suitable because it obscures access to the patient. Alternatively, the patient may be supported on a heated cushion placed on top of the operating table mattress. This, however, can be a disadvantage because it raises the height of the patient and it may make it more difficult to manipulate the patient and use table accessories, such as limb supports and transfer trolleys.

According to one aspect of the present invention there is provided a mattress assembly of the above-specified kind, characterised in that the mattress has at least one fluid passage extending therethrough between an inlet and an outlet, and that the assembly includes a heat exchange unit connected to the inlet, the heat exchange unit being adapted to supply air to the inlet at a selected temperature such that the mattress can be maintained at a required temperature.

The fluid passage is preferably provided by a tube embedded in the foam. The mattress may be of a self-skinning foam, the outer surface of the mattress being provided by a skin on the foam. The fluid passage may have a serpentine shape and the outlet of the mattress may be connected to an inlet of the heat exchanger.

According to another aspect of the present invention there is provided a patient support table having a patient support platform mounted at the upper end of a support column and a mattress assembly according to the above one aspect of the invention.

According to a further aspect of the present invention there is provided a patient support table having a patient support platform mounted at the upper end of a support column and a mattress assembly having a foam mattress supported on a platform, characterised in that the assembly includes a heat exchange unit, that the mattress has at least one fluid passage extending therethrough between an inlet and an outlet, and that the heat exchange unit is connected to the inlet and is adapted to supply air to the inlet at a selected temperature such that the mattress can be maintained at a required temperature.

The support platform is preferably a rigid plate. The table preferably includes three mattresses supported on respective support platforms for supporting the head, body and legs of the patient respectively, only the body mattress having the fluid passage extending therethrough, and the upper surfaces of each mattress being level with one another.

A surgical operating table including a mattress assembly according to the present invention, will now be described, by way of example, with reference to the accompanying drawings, in which:
- Figure 1: is a side elevation view of the table;
- Figure 2: is a cross-sectional plan view of the table mattress to a larger scale; and
- Figure 3: is an sectional side elevation view of a part of the mattress along the line III-III of Figure 2.

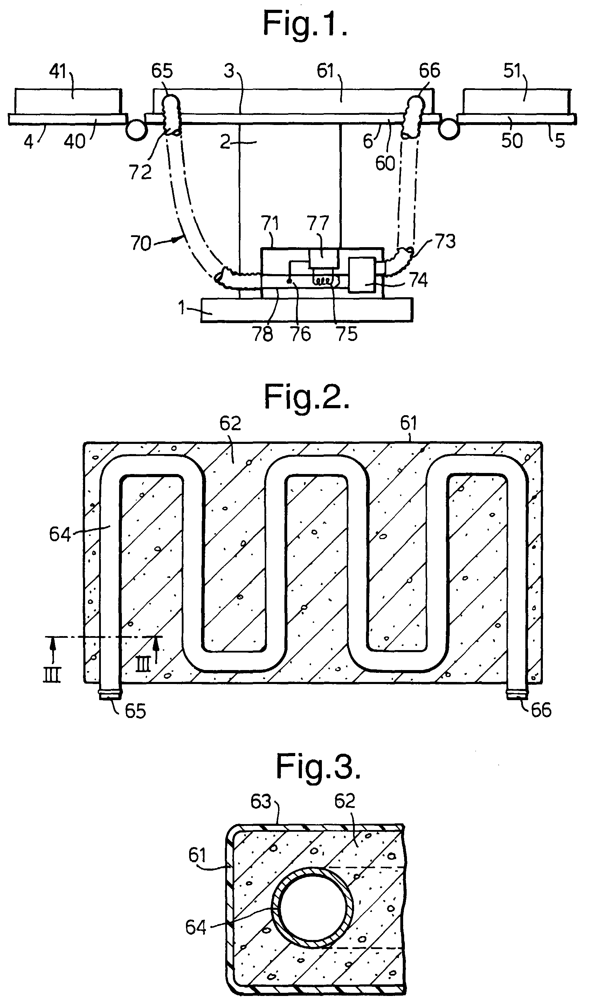

With reference first to Figure 1, the table includes a base 1 with a support column 2, which can be adjusted in height in the usual way. The upper end of the support column 2 supports a patient support platform 3 comprising three sections: a head section 4, a leg section 5 and a main body section 6. The three sections 4 to 6 can be adjusted and locked at different angles relative to one another and to the column 2 in the usual way. The means by which the sections 4 to 6 are adjusted and locked are not illustrated since they are entirely conventional and are not necessary to understand the present invention.

The three sections 4 to 6 each have a metal plate 40, 50 and 60 respectively to which a mattress 41, 51 and 61 is removably secured. The head and leg section mattresses 41 and 51 are conventional foam mattresses. The main body section mattress 61 has a novel construction as described below with reference also to Figure 2 and 3.

The main body section mattress 61 is of rectangular shape being about 100cm long by 80cm wide and 10cm thick. The mattress is made of a foam material 62, which is selfskinning so that it has a smooth, impervious outer skin 63 without joints. The foam material 62 is relatively firm, so that it is self supporting and is not significantly compressed by the weight of the patient. The mattress 61 also has a serpentine tube 64 located midway through the thickness of the mattress and extending backwards and forwards across its width from an inlet 65 to an outlet 66. The inlet 65 is located close to the head end of the mattress 61 on one edge; the outlet 66 is located close to the foot end of the mattress on the same edge. The tube 64 is surrounded and supported by the foam 62 and is of a bendable plastics material having approximately the same resilience as the foam material, so that the upper surface of the mattress has a substantially uniform softness. The tube 64 is of circular section with an external diameter of about 5cm, that is, about half the thickness of the mattress 61. It will be appreciated that other tubes could be used having different shapes and that they could follow different paths within the mattress.

The mattress 61 is a part of a mattress assembly 70, which also includes a heat exchange unit 71, an inlet pipe 72 extending from the mattress inlet 65 to the outlet of the heat exchange unit, and an outlet pipe 73 extending from the outlet 66 of the mattress to the inlet of the heat exchange unit. In the present assembly 70, the heat exchange unit 71 is a heater, although it could be a refrigeration unit, or a unit able both to heat and cool. The heater 71 has an air impeller 74, an electrical heating element 75, a temperature sensor 76 and a control unit 77. The inlet of the impeller 74 is connected to the inlet of the unit 71 and its outlet is connected to an air duct 78 within which the heating element 75 is mounted. The sensor 76 is also mounted in the duct 78 downstream of the heating element 75 so that it provides an output to the control unit 77 representative of the temperature of the heated air. The control unit 77 controls the power supplied to the heating element 75 so that the desired air output temperature is maintained.

In operation, the heater 71 supplies heated air to the mattress inlet pipe 72 so that this flows to the inlet 65 of the tube 64. As the heated air flows along the tube 64, it gives up some of its heat to the wall of the tube and this is conducted by the foam material 62 to the outer surface of the mattress 61 and, more particularly, to the upper surface of the mattress, thereby warming the patient.

Because heating is provided by the mattress of the operating table itself, rather than by a separate cushion placed on top of the mattress, the patient can be supported directly on the mattress without altering the effective height of the table. This is an advantage because it enables existing accessories to be used without modification and, for example, enables patient transfer trolleys to be used easily. It also avoids introducing a difference in height between the upper surfaces of the central, body mattress 61 and the head and leg mattresses 41 and 51. The mattress can be used, without a heater, if heating is not needed. Also, the same mattress can be used for either heating or cooling a patient, by connection to a suitable heat exchange unit. The hospital using such a mattress assembly does not, therefore, need to stock separate mattresses and heating and cooling cushions. The mattress does not present an electrical hazard to the patient because the heat exchanger is located outside the mattress and is only connected to the mattress by air lines, which can be electrically non-conducting. By contrast, if an electrical heating element where placed directly in the mattress, there would be a greater risk of electrocution, especially if the mattress should become damaged. Because air has a relatively low thermal capacity, it will take some time for the mattress to be warmed or cooled to its desired temperature but temperature fluctuations will be relatively slow. Also, if the heat exchange unit should fail, there would be no risk of the patient losing significant heat to the mattress, as could be the case with a liquid-filled mattress. The foam of the mattress is self-supporting, that is, it is not inflated by pressure from the heat exchange unit so that the mattress does not collapse when it is not connected to the heat exchanger, or if the heat exchanger should fail.

The mattress can be made easily at a cost not significantly greater than a conventional foam mattress. This is done by laying the tube in the mattress mould before injecting the foam material. After the foam has been injected and has set, it secures the tube in place.

Various modifications are possible. Any one or more of the three mattresses on the table could include a tube extending through it for the passage of heating/cooling air. These tubes could be connected separately to the heat exchange unit or be interconnected to one another so that heating or cooling air flows from one mattress to another. The mattress could be divided up into different heating/cooling zones, each with its own air tube and inlet and outlet, so that it is possible to control the temperature of the different zones separately. The amount of heat transfer at different regions on the mattress could be altered by, for example, arranging for the turns of the tube to be more closely spaced at some regions than at others, or by varying the diameter of the tube along its length. The outlet of the mattress need not necessarily be connected back to the inlet of the heat exchange unit, it could instead simply vent to atmosphere.

## Claims

1. A mattress assembly (70) including a foam mattress (61) for a patient support table, characterised in that the mattress (61) has at least one fluid passage (64) extending therethrough between an inlet (65) and an outlet (66), and that the assembly (70) includes a heat exchange unit (71) connected to the inlet (65), the heat exchange unit (71) being adapted to supply air to the inlet (65) at a selected temperature such that the mattress (61) can be maintained at a required temperature.

2. A mattress assembly according to Claim 1, characterised in that the fluid passage is provided by a tube (64) embedded in the foam (62).

3. A mattress assembly according to Claim 1 or 2, characterised in that the mattress (61) is of a self-skinning foam (62), and that the outer surface of the mattress is provided by a skin on the foam (62).

4. A mattress assembly according to any one of the preceding claims, characterised in that the fluid passage (64) has a serpentine shape.

5. A mattress assembly according to any one of the preceding claims, characterised in that the outlet (66) of the mattress (61) is connected to an inlet of the heat exchanger (71).

6. A patient support table having a patient support platform (4, 5, 6) mounted at the upper end of a support column (2) and a mattress assembly (70) according to any one of the preceding claims.

7. A patient support table having a patient support platform (4, 5, 6) mounted at the upper end of a support column (2) and a mattress assembly (70) having a foam mattress (61) supported on a platform, characterised in that the assembly (70) includes a heat exchange unit (71), that the mattress (61) has at least one fluid passage (64) extending therethrough between an inlet (65) and an outlet (66), and that the heat exchange unit (71) is connected to the inlet (65) and is adapted to supply air to the inlet (65) at a selected temperature such that the mattress (61) can be maintained at a required temperature.

8. A patient support table according to Claim 7, characterised in that the support platform is a rigid plate (4, 5, 6).

9. A patient support table according to Claim 7 or 8 including three mattresses (41, 51, 61) supported on respective support platforms (4, 5, 6) for supporting the head, body and legs of the patient respectively, characterised in that only the body mattress (61) has the fluid passage (64) extending therethrough, and that the upper surfaces of each mattress (41, 51, 61) is level with one another.
